# EUROPÄISCHE PATENTSCHRIFT

(11) **EP 0 680 947 B1**
(45) Veröffentlichungstag und Bekanntmachung des Hinweises auf die Patenterteilung: **09.09.1998**
(21) Anmeldenummer: 95106068.0
(22) Anmeldetag: 24.04.1995
(51) Int. Cl.: C07C 253/30

(54) **Verfahren zur Herstellung von optischen Aufhellern auf Stilbenbasis**
Process for the preparation of optical brighteners on the basis of stilbenes
Procédé pour la préparation d'azurants optiques à base de stilbènes

(30) Priorität: 02.05.1994 DE 4415351
(43) Veröffentlichungstag der Anmeldung: 08.11.1995
(73) Patentinhaber: BASF AKTIENGESELLSCHAFT, 67056 Ludwigshafen (DE)
(72) Erfinder: Seybold, Günther, Dr., D-67141 Neuhofen (DE); Schmidt, Helmut, Dr., D-67069 Ludwigshafen (DE); Schäfer, Gerhard, Dr., D-69124 Heidelberg (DE); Reichelt, Helmut, Dr., D-67435 Neustadt (DE); Hauptreif, Manfred, Dr., D-76831 Birkweiler (DE); Raatz, Peter, Dr., D-67069 Ludwigshafen (DE); Delavier, Paul, Dr., D-67061 Ludwigshafen (DE)

(56) Entgegenhaltungen:
- EP-A- 0 030 917
- EP-A- 0 032 254
- EP-A- 0 064 303
- GB-A- 920 988
- DEHMLOW E.V. ET AL. 'PHASE TRANSFER CATALYSIS, 2nd Ed.' 1983 , VERLAG CHEMIE , WEINHEIM * Seite 187 - Seite 195 *
- L.F.Fieser und M.Fieser,Organische Chemie 2e Auflage Seite 510
- S.Patai:The chemistry of the carbonyl group(1966)Seite 537
- Organic Reactions, Vol. II, Seiten 103-105, 110

## Beschreibung

Die vorliegende Erfindung betrifft ein neues Verfahren zur Herstellung von Bis(cyanostyryl)benzolen durch Umsetzung von Terephthalaldehyd mit Cyanobenzylphosphonsäuredialkylestern in Gegenwart einer Base und eines Verdünnungsmittels.

Aus der EP-A-32 254 und EP-A-64 303 ist die Herstellung von Bis(cyanostyryl)benzolen bekannt, wobei als Reaktionspartner die oben erwähnten Produkte dienen. Als Verdünnungsmittel kommen Ester, z.B. Methylglykolacetat, und als Base Natriummethanolat zur Anwendung.

Es hat sich jedoch gezeigt, daß die Anwendung von Natriummethanolat als Base nachteilig ist, da in seiner Gegenwart häufig Nebenprodukte gebildet werden, die die anwendungstechnischen Eigenschaften der als optischen Aufheller zur Anwendung gelangenden Bis(cyanostyryl)benzole in ungünstiger Weise beeinflussen.

E.V. Dehmlow et al. "Phase Transfer Catalysis", Verlag Chemie 1983, Seiten 187 bis 194, beschreibt die Umsetzung von aromatischen Carbonylverbindungen in Gegenwart einer Base und eines Phasentransferkatalysators in einem zweiphasigen System, bestehend aus Wasser und einem mit Wasser nicht mischbaren organischen Lösungsmittel, mit Phosphonsäureestern zu ungesättigten Verbindungen.

Weiterhin ist aus der EP-A-30 917 die Herstellung von Bis(cyanostyryl)benzolen durch Umsetzung von aromatischen Aldehyden mit Cyanobenzylphosphonsäureestern bekannt.

Aufgabe der vorliegenden Erfindung war es nun, ein neues Verfahren zur Herstellung von Bis(cyanostyryl)benzolen bereitzustellen, das die genannten Nachteile nicht mehr aufweist.

Es wurde nun gefunden, daß die Herstellung von Stilbenen der Formel I 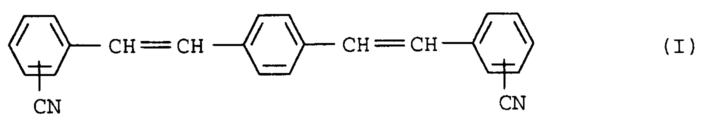 durch Umsetzung von Terephthalaldehyd mit Phosphonsäureestern der Formel II 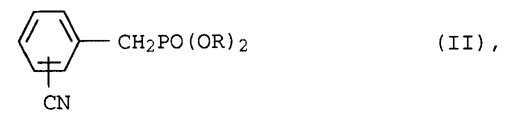 worin R C₁-C₆-Alkyl bedeutet, in einem Verdünnungsmittel in Gegenwart einer Base vorteilhaft gelingt, wenn man als Verdünnungsmittel ein zweiphasiges System verwendet, dessen eine Phase Wasser und dessen andere Phase ein mit Wasser nicht mischbares organisches Lösungsmittel ist, und die Umsetzung in Gegenwart eines Phasentransferkatalysators vornimmt.

Alle in der obengenannten Formel II auftretenden Alkylreste R können sowohl geradkettig als auch verzweigt sein.

Reste R sind z.B. Methyl, Ethyl, Propyl, Isopropyl, Butyl, Isobutyl, sec-Butyl, tert-Butyl, Pentyl, Isopentyl, Neopentyl, tert-Pentyl, Hexyl oder 2-Methylpentyl.

Geeignete mit Wasser nicht mischbare organische Lösungsmittel sind insbesondere aliphatische oder aromatische Kohlenwasserstoffe, wie Pentan, Hexan, Heptan, Octan, Isooctan, Petrolether, Toluol, Xylol, Ethylbenzol, Tetralin, Decalin, Dimethylnaphthalin, Testbenzin, Chlorbenzol oder Dichlorbenzol.

Die Anwendung von aromatischen Kohlenwasserstoffen, insbesondere von Xylol, ist bevorzugt.

Geeignete Basen sind z.B. Alkali- oder Erdalkalimetallhydroxide oder -carbonate, wie Lithium-, Natrium-, Kalium-, Magnesium- oder Calciumhydroxid oder -carbonat.

Besonders zu nennen sind Alkalihydroxide, insbesondere in Form ihrer wäßrigen Lösungen, wobei 25 bis 50 gew.-%ige Natronlauge besonders hervorzuheben ist.

Geeignete Phasentransferkatalysatoren sind alle allgemein üblichen Produkte dieser Art, wie sie z.B. in Ullmann's Encyclopedia of Industrial Chemistry, 5th Edition, Vol. A 19, Seiten 293 bis 298, 1991, beschrieben sind.

Besonders zu nennen sind beispielsweise quartäre Ammoniumsalze, wie Tetraalkylammoniumsalze, insbesondere die entsprechenden Chloride oder Bromide, z.B. Methyltributylammoniumchlorid, Tetrabutylammoniumbromid, Methyltrioctylammoniumchlorid, Methyltricaprylammoniumchlorid, Benzyltrimethylammoniumchlorid oder Benzyltriethylammoniumchlorid.

Bevorzugt ist die Verwendung von Tetraalkylammoniumchloriden oder -bromiden, wobei Tetrabutylammoniumbromid oder Methyltrioctylammoniumchlorid besonders zu nennen ist.

Je mol Terephthalaldehyd kommen in der Regel 1 bis 5 Mol-äquivalent, vorzugsweise 1,2 bis 4 Moläquivalent, Base zur Anwendung.

Bezogen auf das Gewicht von Terephthalaldehyd und Phosphonsäureester II verwendet man üblicherweise 100 bis 200 Gew.-%, vorzugsweise 140 bis 160 Gew.-%, an Verdünnungsmittel.

Erfindungsgemäß wird als Verdünnungsmittel ein zweiphasiges System verwendet. In diesem zweiphasigen System liegen im allgemeinen Wasser und organisches Lösungsmittel im Volumenverhältnis 1:1 bis 1:5, vorzugsweise 1:1,5 bis 1:3, vor.

Bezogen auf das Gewicht an Verdünnungsmittel verwendet man in der Regel 0,3 bis 1,0 Gew.-%, vorzugsweise 0,5 bis 0,8 Gew.-%, an Phasentransferkatalysator.

Das erfindungsgemäße Verfahren wird im allgemeinen bei einer Temperatur von 0 bis 50°C, vorzugsweise 10 bis 35°C, vorgenommen.

In den Stilbenen der Formel I kann die Cyanogruppe entweder in ortho-, meta- oder para-Position zur CH=CH-Gruppe stehen. Dabei können symmetrische oder unsymmetrische Stilbene der Formel I oder auch Mischungen solcher Produkte auftreten.

Zu Mischungen unsymmetrischer Stilbene gelangt man beispielsweise, indem man den Terephthalaldehyd zunächst mit einem ortho-, meta- oder para-cyanosubstituierten Phosphonsäureester der Formel II und anschließend mit einem anderen Isomeren des Phosphonesters II umsetzt.

Zur Herstellung symmetrischer Stilbene der Formel I wird das neue Verfahren zweckmäßig so durchgeführt, daß man Wasser, organisches Lösungsmittel, Phasentransferkatalysator, Base und Phosphonsäureester vorlegt und bei der obengenannten Temperatur unter Rühren Terephthalaldehyd zudosiert. Nach einer Nachrührphase von 2 bis 20 Stunden ist die Umsetzung im allgemeinen beendet und man kann das symmetrische Stilben, gegebenenfalls nach Ansäuern, absaugen, waschen und trocknen.

Zur Herstellung von Mischungen unsymmetrischer Stilbene der Formel I wird das neue Verfahren zweckmäßig so durchgeführt, daß man Wasser, organisches Lösungsmittel, Phasentransferkatalysator, Terephthalaldehyd und, bezogen auf die Menge an Terephthalaldehyd, eine Teilmenge an einem bestimmten Phosphonsäureester der Formel II vorlegt und bei der obengenannten Temperatur unter Rohren eine Teilmenge der Base über einen Zeitraum von 2 bis 8 Stunden zudosiert. Nach einer Nachrührphase von 1 bis 6 Stunden wird dann die restliche Teilmenge an Phosphonsäureester (in Form eines anderen Isomeren) zugegeben und danach die restliche Teilmenge der Base unter Rühren über einen Zeitraum von 2 bis 8 Stunden hinweg zudosiert. Nach einer Nachrührphase von 1 bis 8 Stunden kann das Reaktionsgemisch, wie oben beschrieben, aufgearbeitet werden.

Das neue Verfahren, das sowohl in kontinuierlicher als auch diskontinuierlicher Arbeitsweise vorgenommen werden kann, liefert die Zielprodukte in hoher Ausbeute und Reinheit. Dabei wird die Anwendung von Alkalialkoholaten vermieden. Das zur Anwendung gelangende organische Lösungsmittel kann leicht abgetrennt und wiederverwendet werden.

Wie oben bereits ausgeführt, handelt es sich bei den Stilbenen der Formel I um wertvolle optische Aufheller, insbesondere für Polyester.

Die folgenden Beispiele sollen die Erfindung näher erläutern.

### Beispiel 1

1200 ml Xylol, 620 ml Wasser, 10 g Phasentransferkatalysator auf Basis von Methyltrioctylammoniumchlorid, 793,15 g (3,00 mol) o-Cyanobenzylphosphonsäurediethylester (95,7 gew.-%ig) und 290 g (3,63 mol) 50 gew.-%ige Natronlauge wurden bei 30°C vorgelegt. Anschließend dosierte man bei dieser Temperatur unter Rühren innerhalb von 3,5 h 249,1 g (1,67 mol) Terephthalaldehyd (89 gew.-%ig) zu, rührte zunächst 2 h bei 30°C, dann 2 h bei 20°C nach. Der Niederschlag wurde abgesaugt und das Nutschgut zunächst mit 1000 ml Xylol und anschließend mit 1500 ml Wasser nachgewaschen. Den Rückstand trocknete man unter vermindertem Druck bei 60°C.

Man erhielt 420 g (84 %) der Verbindung der Formel

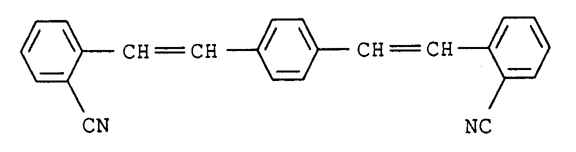

### Beispiel 2

1000 ml Xylol, 350 ml Wasser, 10 g Phasentransferkatalysator auf Basis von Methyltrioctylammoniumchlorid, 431,7 g (1,58 mol) o-Cyanobenzylphosphonsäurediethylester (92 gew.-%ig) und 208,2 g (1,50 mol) Terephthalaldehyd (96,4 gew.-%ig) wurden bei 35°C vorgelegt. Anschließend dosierte man bei dieser Temperatur zunächst 148,8 g (0,93 mol) 25 gew.-%ige Natronlauge innerhalb von 4 h, dann 99,2 g (0,62 mol) 25 gew.-%ige Natronlauge innerhalb von 4 h zu und rührte anschließend 2 h nach. 405,7 g (1,50 mol) m-Cyanobenzylphosphonsäurediethylester (93,1 gew.-%ig) wurden zugegeben. Zum Ansatz fügte man dann innerhalb von 1 h bei 35°C 640 g (4 mol) 25 gew.-%ige Natronlauge zu und rührte 8 h bei dieser Temperatur nach. Es wurde auf Raumtemperatur abgekühlt, 1 h bei dieser Temperatur gerührt und der Niederschlag abgesaugt. Das Nutschgut wusch man zunächst mit 1500 ml Methanol und anschließend mit 1650 ml Wasser. Den Rückstand trocknete man unter vermindertem Druck bei 60°C.

Man erhielt 334 g (67 %) einer Mischung, enthaltend 82,3 % der Verbindung der Formel 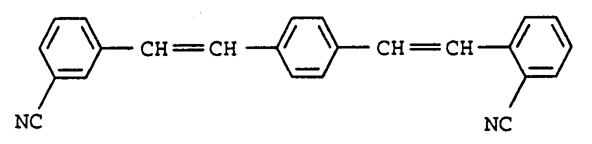 und 9,3 % der Verbindung der Formel

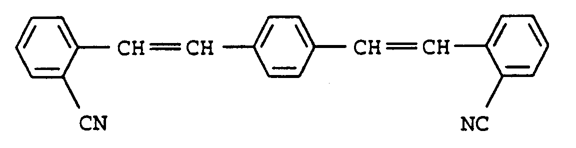

### Beispiel 3

1000 ml Xylol, 350 ml Wasser, 10 g Phasentransferkatalysator auf Basis von Methyltrioctylammoniumchlorid, 431,7 g (1,58 mol) o-Cyanobenzylphosphonsäurediethylester (92 gew.-%ig) und 208,2 g (1,50 mol) Terephthalaldehyd (96,4 gew.-%ig) wurden bei 35°C vorgelegt. Anschließend dosierte man bei dieser Temperatur zunächst 148,8 g (0,93 mol) 25 gew.-%ige Natronlauge innerhalb von 4 h, dann 99,2 g (0,62 mol) 25 gew.-%ige Natronlauge innerhalb von 4 h zu und rührte anschließend 2 h nach. 407,2 g (1,50 mol) p-Cyanobenzylphosphonsäurediethylester (92,7 gew.-%ig) wurden zugegeben. Zum Ansatz fügte man dann innerhalb von 1 h bei 35°C 640 g (4 mol) 25 gew.-%ige Natronlauge zu und rührte 8 h bei dieser Temperatur nach. Es wurde auf Raumtemperatur abgekühlt, 1 h bei dieser Temperatur gerührt und der Niederschlag abgesaugt. Das Nutschgut wusch man zunächst mit 1500 ml Methanol und anschließend mit 1650 ml Wasser. Den Rückstand trocknete man unter vermindertem Druck bei 60°C.

Man erhielt 354 g (71 %) einer Mischung, enthaltend 87 % der Verbindung der Formel 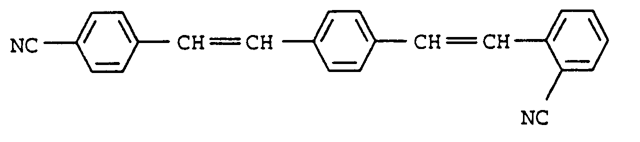 und 8,3 % der Verbindung der Formel

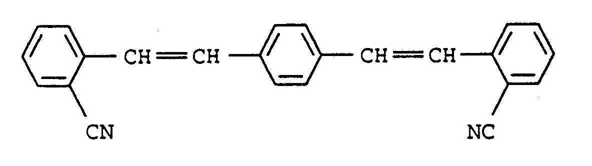

### Beispiel 4

1000 ml Xylol, 350 ml Wasser, 10 g Phasentransferkatalysator auf Basis von Methyltrioctylammoniumchlorid, 429,4 g (1,58 mol) m-Cyanobenzylphosphonsäurediethylester (93,1 gew.-%ig) und 208,2 g (1,50 mol) Terephthalaldehyd (96,4 gew.-%ig) wurden bei 35°C vorgelegt. Anschließend dosierte man bei dieser Temperatur zunächst 148,8 g (0,93 mol) 25 gew.-%ige Natronlauge innerhalb von 4 h, dann 99,2 g (0,62 mol) 25 gew.-%ige Natronlauge innerhalb von 4 h zu und rührte abschließend 2 h nach. 420,3 g (1,50 mol) p-Cyanobenzylphosphonsäurediethylester (90,3 gew.-%ig) wurden zugegeben. Zum Ansatz dosierte man dann innerhalb von 1 h bei 35°C 640 g (4 mol) 25 gew.-%ige Natronlauge und rührte 8 h bei dieser Temperatur nach. Es wurde auf Raumtemperatur abgekühlt, 1 h bei dieser Temperatur gerührt und der Niederschlag abgesaugt. Das Nutschgut wusch man zunächst mit 1500 ml Methanol und anschließend mit 1650 ml Wasser. Den Rückstand trocknete man unter vermindertem Druck bei 60°C.

Man erhielt 342 g (69 %) einer Mischung, enthaltend 80 % der Verbindung der Formel 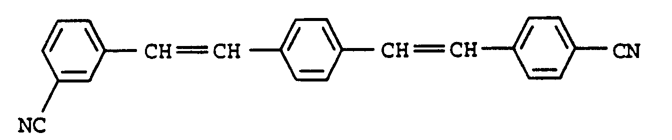 und 6 % der Verbindung der Formel

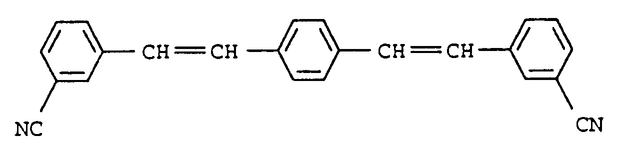

### Beispiel 5

Zu 1 Liter Chlorbenzol wurden 660 g (2,37 mol) o-Cyanobenzylphosphonsäurediethylester gegeben und die Mischung anschließend mit 500 ml Wasser sowie 12 g Phasentransferkatalysator auf Basis von Methyltrioctylammoniumchlorid versetzt. Dann wurden langsam 580 g 25 gew.-%ige Natronlauge zugetropft, wobei die Temperatur auf ca. 35°C anstieg. Danach wurden innerhalb von 2 h 167 g (1,185 mol) Terephthalaldehyd eingetragen, wobei die Innentemperatur zwischen 35 und 40°C gehalten wurde. Nach vollendeter Zugabe wurde noch 4 h bei dieser Temperatur gerührt, anschließend 110 g konz. Schwefelsäure zugetropft.

Der Niederschlag wurde abgesaugt und mit 600 ml Chlorbenzol gewaschen. Anschließend wurde Chlorbenzol mit Dampf ausgeblasen und der Filterkuchen mehrmals mit Wasser gewaschen.

Man erhielt 314 g (80 %) hellgelbes, kristallines Produkt der Formel 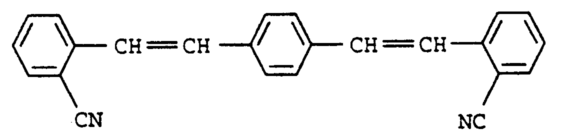 (Fp: 221 bis 225°).

## Patentansprüche

1. Verfahren zur Herstellung von Stilbenen der Formel I 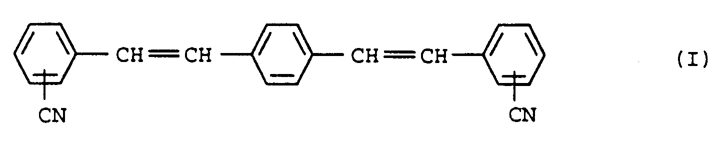 durch Umsetzung von Terephthalaldehyd mit Phosphonsäureestern der Formel II 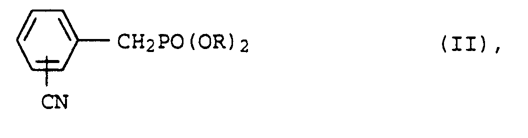 worin R C₁-C₆-Alkyl bedeutet, in einem Verdünnungsmittel in Gegenwart einer Base, dadurch gekennzeichnet, daß man als Verdünnungsmittel ein zweiphasiges System verwendet, dessen eine Phase Wasser und dessen andere Phase ein mit Wasser nicht mischbares organisches Lösungsmittel ist, und die Umsetzung in Gegenwart eines Phasentransferkatalysators vornimmt.

2. Verfahren nach Anspruch 1, dadurch gekennzeichnet, daß man die Umsetzung bei einer Temperatur von 0 bis 50°C vornimmt.

3. Verfahren nach Anspruch 1, dadurch gekennzeichnet, daß man als Base ein Alkali- oder Erdalkalimetallhydroxid oder -carbonat verwendet.

4. Verfahren nach Anspruch 1, dadurch gekennzeichnet, daß man als Verdünnungsmittel ein zweiphasiges System verwendet, in dem Wasser und mit Wasser nicht mischbares organisches Lösungsmittel im Gewichtsverhältnis 1:1 bis 1:5 vorliegen.

5. Verfahren nach Anspruch 1, dadurch gekennzeichnet, daß man als Verdünnungsmittel ein zweiphasiges System aus Wasser und einem aromatischen Kohlenwasserstoff verwendet.

6. Verfahren nach Anspruch 1, dadurch gekennzeichnet, daß man als Phasentransferkatalysator ein Tetraalkylammoniumchlorid oder -bromid verwendet.

## Claims

1. A process for preparing stilbenes of the formula I 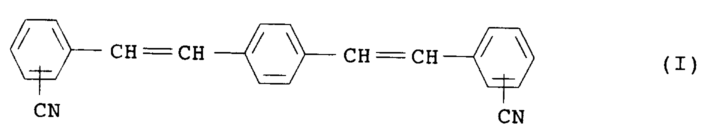 by reacting terephthalaldehyde with phosphonic esters of the formula II 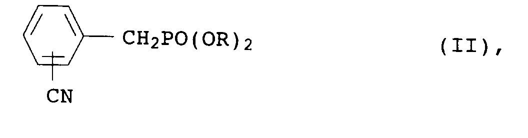 where R is C₁-C₆-alkyl, in a diluent in the presence of a base, wherein the diluent used is a two-phase system, one phase of which is water and the other phase of which is a water-immiscible organic solvent, and the reaction is carried out in the presence of a phase transfer catalyst.

2. A process as claimed in claim 1, wherein the reaction is carried out at from 0 to 50°C.

3. A process as claimed in claim 1, wherein an alkali metal or alkaline earth metal hydroxide or carbonate is used as base.

4. A process as claimed in claim 1, wherein the diluent used is a two-phase system in which water and water-immiscible organic solvent are present in the ratio from 1:1 to 1:5 by weight.

5. A process as claimed in claim 1, wherein a two-phase system composed of water and an aromatic hydrocarbon is used as diluent.

6. A process as claimed in claim 1, wherein a tetraalkylammonium chloride or bromide is used as phase transfer catalyst.

## Revendications

1. Procédé de préparation de stilbènes de formule I 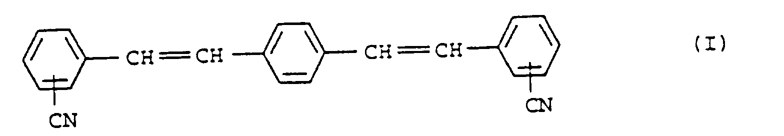 par réaction d'aldéhyde téréphtalique avec des esters d'acide phosphonique de formule II 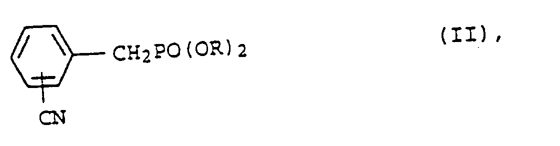 dans laquelle R représente un groupement alkyle en C₁-C₆, dans un diluant en présence d'une base, caractérisé en ce que l'on utilise comme diluant un système biphasique dont une phase est constituée d'eau et l'autre phase est un solvant organique non miscible à l'eau, et en ce que la réaction a lieu en présence d'un catalyseur de transfert de phases.

2. Procédé selon la revendication 1, caractérisé en ce que l'on effectue la réaction à une température de 0-50°C.

3. Procédé selon la revendication 1, caractérisé en ce que l'on utilise en tant que base un carbonate ou un hydroxyde de métal alcalin ou alcalino-terreux.

4. Procédé selon la revendication 1, caractérisé en ce que l'on utilise comme diluant un système biphasique dans lequel l'eau et le solvant organique non miscible à l'eau se trouvent dans un rapport en poids de 1:1 à 1:5.

5. Procédé selon la revendication 1, caractérisé en ce que l'on utilise comme diluant un système biphasique constitué d'eau et d'un hydrocarbure aromatique.

6. Procédé selon la revendication 1, caractérisé en ce que l'on utilise comme catalyseur de transfert de phases un chlorure ou un bromure de tétraalkylammonium.
